Europäisches Patentamt

European Patent Office

Office européen des brevets

(11) Publication number: **0 271 241**
**A1**

## (12) EUROPEAN PATENT APPLICATION

(21) Application number: **87310239.6**

(22) Date of filing: **19.11.87**

(51) Int. Cl.⁴ **A61F 5/44**

(30) Priority: **20.11.86 GB 8627732**

(43) Date of publication of application:
**15.06.88 Bulletin 88/24**

(84) Designated Contracting States:
**AT BE CH DE ES FR GB GR IT LI LU NL SE**

(71) Applicant: **H.G. Wallace Limited**
**Whitehall Road**
**Colchester Essex CO2 8JH(GB)**

(72) Inventor: **Wallace, Henry George**
**"Treetops", First Avenue**
**Frinton-on-Sea, Essex(GB)**

(74) Representative: **Sanderson, Laurence Andrew**
**et al**
**SANDERSON & CO. European Patent**
**Attorneys 34, East Stockwell Street**
**Colchester Essex CO1 1ST(GB)**

(54) **Fluid collection assemblies.**

(57) A bag assembly for collecting fluids discharged from a patient comprises a bag (10) and at least one strap (16) connected to the bag (10) and adapted to secure the bag to a limb of the patient. The strap (16) is made of an elasticated fabric and carries on the face thereof intended to contact the patient's limb at least one bead (22) of a silicone rubber material. The two end portions (17,19) of the strap (16) carry the respective components (18,20) of a two-component hook-and-loop fastener to allow the two end portions to be fastened together in an adjustable manner.

FIG.1

## FLUID COLLECTION ASSEMBLIES

This invention relates to a bag assembly for collecting fluids discharged from a human patient, the bag assembly being adapted for mounting on a limb of the patient so that the bag does not limit the mobility of the patient.

Not infrequently, it is necessary to collect a body fluid involuntarily discharged from a human body and for this purpose it is known to utilise a flexible bag having an inlet duct connected for example a catheter adapted to drain the body fluid, such as urine. In order to minimise the inconvenience to the patient, such a bag may be mounted on a limb of the patient, for example by means of two straps which are tightened around the limb. However, significant problems are encountered in providing a satisfactory strap arrangement.

When a bag is to be mounted on a leg by straps, the tapering shape of the leg and the weight of discharged fluid results in a tendency for the straps to slip downwardly.

Rubber latex straps are commonly employed for securing a bag to the limb of a patient, for such a material has good grip and elasticity characteristics. Nevertheless, such straps are prone to roll along their length into a string-like configuration, causing constrictive tension upon tissues and blood circulation. Also, rubber latex can cause irritation, especially if perspiration is present, for the rubber latex material prevents aeration of the skin. Studs are normally provided adjustably to secure together the end portions of a strap, but these can cause pressure on tissues.

These problems can to some extent be reduced by providing elasticated straps, but the surfaces of such straps lack good grip qualities and if too much tension is used in the straps, blood circulation may be restricted. Providing two tracks of rubber stitched into the elasticated straps along their length is an improvement, but allows exposed and raised rubber thread to irritate the skin by nature of the rubber material and its abrasive action on the skin. Also, sensitivity of human skin to long-term contact with certain materials limits the choice of materials suitable for manufacturing the straps. Moreover, elderly people - who more frequently than others have to wear such devices - often have skin which is fragile and easily broken, and thus the use of straps can cause especial difficulties.

There have been other proposals for alternatives to rubber latex straps but so far none has proved wholly satisfactory. For example, sponge-lined non-elastic straps are sometimes used, though these may constrict when tight but slip when loosened for comfort. The sponge also absorbs perspiration resulting in odour, and the straps cannot easily be washed and dried.

It is a principal aim of the present invention to provide a bag assembly including a mounting arrangement for mounting a fluid-receiving bag on the limb of a patient, which overall assembly at least mitigates some of the disadvantages discussed above, for the known assemblies.

According to this invention, a bag assembly for collecting fluid discharged from a human patient and comprising a fluid receiving bag and means to mount the bag on a limb of the patient, the mounting means including at least one elasticated strap connected to the bag and adapted to surround the limb, the strap being provided with adjustment means allowing the effective length of strap to be adjusted, is characterised by the surface of the strap intended to contact the patient's limb having at least one region with a lengthwise extent with respect to the strap which region has one or more areas of an applied silicone rubber material to impart limb-gripping properties to the strap.

It will be appreciated that by using in a bag assembly of the present invention incorporating an elasticated strap having an area of a silicone rubber as defined above, many of the disadvantages mentioned above of the known bag arrangements can largely be overcome. Silicone rubber is a synthetic polymer which is biologically substantially inert. It has excellent gripping qualities but also provides a soft, gentle and non-abrasive surface. Moreover, silicone rubber is non-wettable and cannot therefore absorb odour-inducing perspiration or other fluids.

The said one region of the strap may extend for substantially the whole of the limb-contacting surface of the strap, and at least one continuous area of applied silicone rubber material may be provided within that region. For example, the silicone rubber material may be a continuous rib or bead extending along the length of the strap for essentially the whole of the length thereof, part-way between the side edges of the strap. Two, three or even more such ribs or beads may run in a generally parallel but spaced-apart manner along the length of the strap surface. Though such ribs may be substantially straight, they may instead be curved or angulated, along their length. Other possibilities include a network of ribs or beads for instance of square or diamond shape, arrays of discreet spots of silicone rubber material, or even a series of relatively large patches of silicone rubber spaced along the length of the said one region. Other possibilities will readily suggest themselves to those skilled in this art.

The silicone rubber material may be applied to the strap by any suitable method for attaching such a material to others. For example, it may be directly applied by an extrusion process, preferably so that the rubber material is urged into the surface of the strap, whereby the extruded rubber will not press unduly into the skin of a patient when in use, so minimising, discomfort. Instead, the silicone rubber material may be cast or moulded to the required shape and then affixed to the strap by a heat or chemical welding process or by an adhesive. Another possibility is the stitching of one or more rows of silicone rubber monofilaments or braids into or on to the strap. Whichever method is employed, the arrangement should be such that the stretching of the strap within its limits does not overstretch the silicone rubber material, to avoid cracking of the silicone rubber material.

In a preferred embodiment, the bag is connected to the strap part-way between the ends of the strap, the adjustment means comprising fastening means to secure the end portions of the strap together in an adjustable manner. However, in order to minimise irritation and possible damage to the patient's tissues in the region where the end portions of the strap are fastened together, it is greatly preferred for the fastening means not to have any significant inwardly-directed extent. To this end, it is preferred for the two end portions of the strap to be provided with two-component hook-and-loop pressure fasteners, one component being provided on each end portion respectively. Such pressure fasteners have on one component a mass of upstanding thread-like loops, and on the other component a mass of resilient bristles each having at its free end a hook which may interengage with a loop on the other component when the two components are pressed together; the two components can however easily be separated when desired. One such fastener is for example sold under the Registered Trade Mark **VELCRO.**

When a pressure fastener of the kind described above is used for securing the end portions of the strap together, an elasticised relatively short length of one of the components should be provided on the inwardly-directed surface of one strap end portion, whereas on the other end portion a relatively long length of the other component may be provided, but on the outwardly-directed surface of that end portion. In this way, the strap may be adjusted over a wide range, and yet direct contact with the skin by either one of the two components of the fastener is avoided.

An alternative arrangement is to have the two end portions attached to the bag, at least one end portion being releasable and adjustable with respect to the bag, for example by means of press-stud fasteners or the like.

By way of example only, one specific embodiment of this invention will now be described in detail, reference being made to the accompanying drawings, in which:-

Figure 1 is a general perspective view of a bag assembly of this invention; and

Figure 2 is a cross-section through one of the straps, used in the assembly of Figure 1, at a position marked A-A on that Figure.

Referring to the drawings, a bag assembly of this invention includes a known form of fluid-receiving bag 10 having an inlet nozzle 11 adapted for the connection thereto of a catheter tube. The bag may be provided with a drainage tube and closure as shown at its lower end, if required. The bag is conveniently manufactured from a flexible plastics material and has welded seams 12 to ensure that the bag is fluid tight, apart from its inlet duct and drainage duct, if provided.

The welded seams adjacent the upper end of the bag define two closed-off areas 13 and 14, through each of which is provided an aperture 15. A strap 16 is passed through these two apertures 15, so that on securing the strap around the limb of a patient, the bag may securely be mounted on that limb. Depending upon the size of the bag, a similar arrangement with a second strap may be provided at the lower end of the bag, as shown.

The strap 16 is manufactured from an elasticated fabric which is porous, so as to minimise problems which may arise from the patient perspiring locally, beneath the strap. End portion 17 of the strap is provided with one component 18 of a two-component hook-and-loop pressure fastener, this component being elasticated and secured on the surface of the strap which is intended to contact the skin of a patient. The other end portion 19 is provided with the other component 20 of the fastener, on the surface of that end portion which is intended to be directed outwardly of the limb. Preferably, said other component 20 extends along the strap for a considerably greater distance than does the one component 18 on end portion 17; in this way a large range of adjustment may be obtained without risk of contact with the skin by either component of the fastener.

One example of a two component fastener of the kind described above is sold under the Registered Trade Mark **VELCRO.**

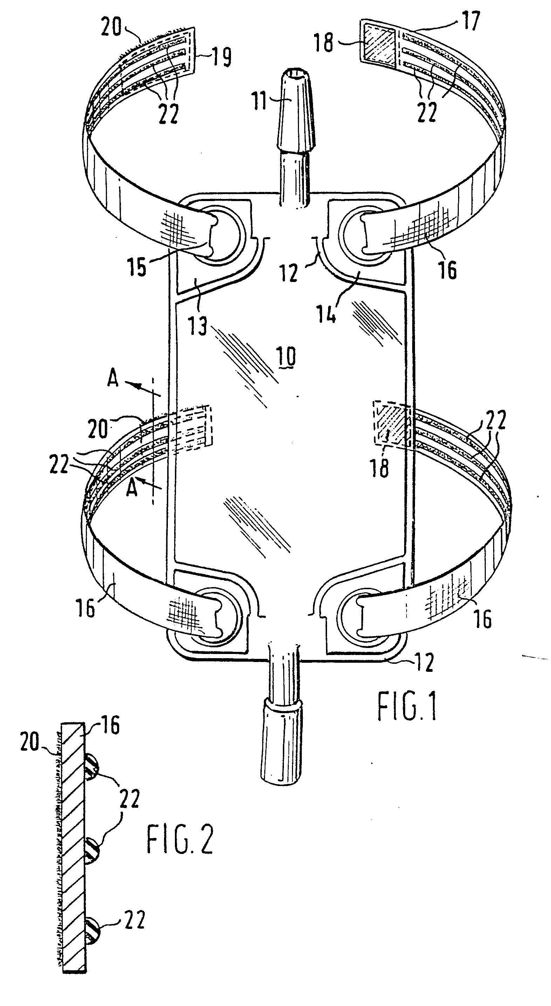

Extending along the inwardly directed surface of the strap which is intended to contact the skin of a patient are three parallel ribs 22, each formed of a silicone rubber material. Each rib should have a rounded profile, as shown in Figure 2, and may be directly extruded on to the fabric of the strap, following the manufacture thereof. The silicone rubber material employed should be relatively soft, and so typically may have a Shore 'A' hardness within the range of from 50 to 60.

In use, the bag 10 can be secured to a limb (such as the leg) of a patient by lightly tensioning the strap 16 around the limb, the ends of the strap being fastened together by pressing fastener component 18 closely to engage the other fastener component 20. The strap then serves securely to hold the bag in the required position, by virtue of the silicone rubber ribs 22 which gently but surely grip the skin. The silicone rubber has a non-abrasive surface and also is non-wettable so that it will not absorb odour-inducing perspiration.

The straps described above may be used with other medical garments besides the illustrated bag. For example, various pouches, bags, so-called 'holsters' or other medical receptacles may be suspended from at least one strap which is adapted to be passed around a limb or the torso of a patient. Also, the straps may be used for orthopaedic suspension, where similar advantages can be obtained to those associated with the present invention.

## Claims

1. A bag assembly for collecting fluid discharged from a human patient, which bag assembly comprises a fluid receiving bag (10) and means to mount the bag on a limb of the patient, the mounting means including at least one elasticated strap (16) connected to the bag and adapted to surround the limb, the strap (16) being provided with adjustment means allowing the effective length of the strap to be adjusted, characterised in that the surface of the strap (16) intended to contact the patient's limb having at least one region with a lengthwise extent with respect to the strap (16) which said one region has one or more areas of an applied silicone rubber material (22) to impart limb-gripping properties to the strap (16).

2. A bag assembly according to claim 1, further characterised in that the said one region extends for substantially the whole of the limb-contacting surface of the strap (16).

3. A bag assembly according to claim 2, characterised in that there is at least one continuous area of applied silicone rubber material (22) provided within said one region.

4. A bag assembly according to any of the preceding claims, characterised in that the silicone rubber material is applied as at least one continuous rib (22) or bead extending along the length of the strap for essentially the whole of the length thereof, part-way between the side edges of the strap (16).

5. A bag assembly according to claim 4, characterised in that two or more ribs or beads of silicone rubber material are provided, which beads are one of substantially straight, curved or angulated along their length

6. A bag assembly according to claim 1 or claim 2, characterised in that the silicone rubber material is applied as a network of ribs or beads, or as arrays of discreet spots or as a series of relatively large patches of silicone rubber spaced along the length of the said one region.

7. A bag assembly according to any of the preceding claims, characterised in that the silicone rubber material is applied by an extrusion process directly on the strap, or is cast or moulded to the required shape and is then affixed to the strap by a heat or chemical welding process or by an adhesive.

8. A bag assembly according to any of claims 1 to 7, characterised in that one or more rows of silicone rubber monofilaments or braids are stitched into or on to the strap.

9. A bag assembly according to any of the preceding claims, characterised in that the two end portions (17,19) of the strap (16) are provided with two-component (18,20) hook-and-loop pressure fasteners, one component being provided on each end portion respectively.

10. A bag assembly according to claim 9, characterised in that an elasticated relatively short length (18) of one of the fastener components is provided on an inwardly-directed surface of one end portion (17), of the strap the other end portion (19) carrying a relatively long length of the other component (20) of the fastener, but on the outwardly-directed surface of that end portion.

FIG.1

FIG.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | DE-A-3 241 517 (KARL) <br> * Figure 1; column 3, lines 5-18,24-30; column 4, lines 37-45 * | 1-5,7,9 | A 61 F   5/44 |
| Y | US-A-3 590 390 (HOWARD) <br> * Figure 2; column 1, line 71 - column 2, line 18; column 2, lines 43,44; column 3, lines 9-34; column 4, lines 4-9 * | 1-5,7,9 | |
| A | WO-A-8 201 648 (HAAN) <br> * Figure 2; page 4, lines 16-19 * | 5,6,10 | |
| A | US-A-3 800 331 (TADDEO) <br> * Figures 2-4; column 3, lines 20-66 * | 8 | |

TECHNICAL FIELDS
SEARCHED (Int. Cl.4)

A 61 F
A 41 B

The present search report has been drawn up for all claims

| Place of search | Date of completion of the search | Examiner |
|---|---|---|
| THE HAGUE | 09-02-1988 | SEDY, R. |